# EUROPEAN PATENT APPLICATION

(11) **EP 4 614 149 A1**
(43) Date of publication of application: **10.09.2025**
(21) Application number: 24161814.9
(22) Date of filing: 06.03.2024
(51) Int. Cl.: G01N 33/564, G01N 33/68, A61K 38/00

(54) **AUTOANTIBODIES AGAINST NAV1.5 CHANNEL AS BIOMARKERS FOR THE DIAGNOSIS OF BRUGADA SYNDROME**

(71) Applicant: Cardiomix S.r.l., 20121 Milano (IT)
(72) Inventor: Anastasia, Luigi, 20121 Milano (IT); Tarantino, Adriana, 20121 Milano (IT); Ghiroldi, Andrea, 20121 Milano (IT); Ciconte, Giuseppe, 20121 Milano (IT); Pappone, Carlo, 20121 Milano (IT)
(74) Representative: Bird & Bird Società tra Avvocati S.r.l.

(57) **Abstract**

The present invention relates to autoantibodies against NaV1.5 channel and related isoforms, their use as biomarker for the diagnosis of Brugada syndrome in human beings and relative methods of detection and therapeutic approach to target this new discovered form of autoimmunity.

## Description

The present invention relates to autoantibodies against NaV1.5 channel and related isoforms, their use as biomarker for the diagnosis of Brugada syndrome in human beings and relative methods of detection and therapeutic approach to target this new discovered form of autoimmunity.

The Brugada Syndrome (BrS) is an inherited arrhythmogenic disease that poses an increased risk of sudden cardiac death (SCD), accounting for 5-40% of SCD cases in individuals under 40 years of age [1]. The syndrome typically manifests with cardiac arrest or syncope, occurring in the third and fourth decade of life [2] [3] however, the majority of patients are asymptomatic with structurally normal heart, and they are usually diagnosed incidentally.

It is diagnosed by the presence of coved-type ST-segment elevation in right precordial leads on an electrocardiogram (ECG), which can manifest spontaneously or after provocative drug testing with intravenous sodium channel blockers [4]. While some patients experience cardiac arrest or syncope in their third and fourth decades of life, the majority of patients are asymptomatic with structurally normal hearts [2]. Brugada syndrome is a complex disorder influenced by both genetic and non-genetic factors [5, 6] involving mutations in more than 23 genes encoding sodium, potassium, and calcium channels, as well as proteins responsible for their trafficking [7].

Among these genes, *SCN5A,* which encodes the alpha subunit of the voltage-gated sodium channel NaV1.5, is the primary causative gene for Brugada syndrome [8]. However, the genetic etiology remains unclear in approximately 70-75% of cases, and a single mutation does not fully account for the Brugada syndrome phenotype [9], rendering genetic testing inadequate as a standalone diagnostic tool for the disease.

Brugada syndrome is not solely driven by genetic factors, as evidenced by histological changes in the right ventricular myocardium of type 1 Brugada patients [10, 11] and the presence of inflammatory infiltrates and fibrosis in the right ventricular outflow tract of Brugada patients has been reported [12, 13]. Furthermore, emerging evidence suggests a potential role of autoimmunity in Brugada syndrome, which has long been overlooked in cardiac arrhythmias. The discovery of autoantibodies associated with arrhythmogenic right ventricular cardiomyopathy (ARVC) has shed light on the autoimmune implications in Brugada syndrome [14, 15].

Autoantibodies can interfere with ion channels and receptors involved in cardiac electrophysiology, triggering arrhythmias. For instance, autoantibodies targeting β1-adrenergic receptors have been reported in various cardiac diseases, including ischemic cardiomyopathy and Chagas' disease [16, 17]. Additionally, IgG antibodies against the voltage-gated KCNQ1 K+ channel (Kv7.1 or KvLQT1) have been detected in patients with dilated cardiomyopathy, resulting in a shortened QTc interval [18]. Autoantibodies targeting the cardiac voltage-gated Na+ channel have also been found in patients with idiopathic high-degree AV block, leading to reduced sodium current (INa) density in rat cardiomyocytes [19].

Overall, there is a need of further investigating the involvement of immune system in Brugada syndrome beyond genetic factors to fully comprehend the disease.

Previous studies have shown abnormal protein distribution in Brugada syndrome myocardium, such as α-actin, keratin-24, and connexin-43, which may affect the trafficking processes of sodium channel complexes [12]. These proteins have also been identified as potential targets of autoantibodies, indicating an abnormal immune response. Therefore, the presence of NaV1.5 channel autoantibodies in Brugada syndrome plasma has not been conclusively demonstrated so far.

The authors of present invention have now addressed the pathophysiology of Brugada syndrome beyond genetic factors by investigating the presence and impact of autoantibodies against the NaV1.5 channel.

In particular, the inventors have developed an in-vitro model of NaV1.5 overexpressing channels with improved purification capabilities and the ability to test autoantibody binding under denaturing and native conditions, mimicking the physiological environment. Previous studies have shown that autoantibodies against NaV1.5 can affect sodium ion currents in vivo in rats [20]. This mechanism aligns with the activity of autoantibodies against channel or receptor proteins found in other diseases, such as NMDAR encephalitis, where autoantibodies promote receptor internalization and induce electrophysiological changes in neurons [21-23]. By targeting autoimmunity, these outbreaking findings hold promise for the development of therapeutic strategies and improved patient care in Brugada syndrome. Mitigating the detrimental effects of autoantibodies on NaV1.5 channels could potentially lead to better management of Brugada syndrome and its associated arrhythmias.

Therefore, it is an object of the present invention antibodies for use as biomarkers for the diagnosis of Brugada Syndrome in a human being directed against the NaV 1.5 channel (SEQ ID NO:1) and related isoforms.

In fact, the autoantibodies targeting NaV1.5 channel (SEQ ID NO: 1) may be cross reactive with other NaV1.5 channel isoforms. Due to the high sequence similarity between the canonical NaV1.5 channel and its isoforms, including the embryonal variant nNaV1.5, autoantibodies initially generated against these alternative isoforms may also interact with the cardiac NaV1.5 channel. Such cross-reactivity can contribute to the pathophysiology of Brugada Syndrome by affecting the functional integrity of the cardiac sodium channel, potentially exacerbating the risk of arrhythmias.

Therefore, according to the present invention the expression "NaV1.5 channel and related isoforms," encompasses any and all variants of the NaV1.5 channel, including but not limited to the embryonal isoform, which share significant sequence homology with the canonical NaV1.5 channel (SEQ ID NO:1). With the expression "significant sequence homology" it is intended a sequence homology greater than 85%, preferably greater than 90%, even more preferably greater than 95%, 96%, 97%, 98% or 99%.

In a preferred embodiment the antibodies for use as biomarkers for the diagnosis of Brugada Syndrome in a human being of the invention are directed against a binding site of the extracellular loops of NaV 1.5 channel (SEQ ID NO:1) and related isoforms.

However, it can not be excluded that in the presence of a misfolded protein channel in Brugada patients the autoantibodies may be directed also against a binding site exposed in the inner channel protein.

Preferably, the antibodies for use as biomarkers for the diagnosis of Brugada Syndrome of the invention are directed against a binding site of the extracellular loops of NaV 1.5 channel and related isoforms, wherein said binding site may be selected from the group comprising the following sequences:
i) VFALIGLQLFMGNLRHKCVRNFTALNGTNGSVEADGLVWESLDLYLS DPENYLLKNGTSDVLLCGNSSDAGTCPEGYRCLKAGENPDHGYTSFDSFAWAFLALF RL (SEQ ID NO:2)
ii) FGKNYSELRDSDSGLLPRWHMMDFFHAFLIIFRILCG (SEQ ID NO:3)
iii) SIMGVNLFAGKFGRCINQTEGDLPLNYTIVNNKSQCESLNLTGELYW TKVKVNFDNVGAGYLALLQ-1414 (SEQ ID NO: 4)
iv) VILSIVGTVLSDIIQKYFFSPTLFRVIRLARIGRIL (SEQ ID NO:5)
v) IYSIFGMANFAYVKWEAGIDDMFNFQTFANSMLCLFQI (SEQ ID NO:6)
vi) AGWDGLLSPILNTGPPYCDPTLPNSNGSRGDCGSPAVGILFFTT (SEQ ID NO:7)
vii) SVEADGLVWESLDLYLSDPENYLLKNGTS (SEQ ID NO:8)
viii) CLKAGENPDHGYTSFDSFAWAFLAL (SEQ ID NO:9)
ix) DSDSGLLPRWHMMDFFHAFLII (SEQ ID NO: 10)
x) VNNKSQCESLNLTGEYWTKVK (SEQ ID NO:11)
xi) GSGVILSIVGTVLSDIIQKYFFSPT (SEQ ID NO:12)
xii) MANFAYVKWEAGIDDMFNFQTFANSMLCLF (SEQ ID NO:13)
xiii) GWDGLLSPILNTGPPYCDPTLPNSNGSRGD (SEQ ID NO:14)
or fragments thereof.

Said antibodies directed against the NaV 1.5 channel (SEQ ID NO:1), preferably against a binding site of the extracellular loops of NaV 1.5 channel, more preferably against a binding site having a sequence selected between SEQ ID NO: 2-14, are autoantibodies depicted in a biological sample of a human being affected by Brugada Syndrome.

The invention further relates to an in vitro method for detecting the presence or detecting the amount of at least one of the antibodies directed against the NaV 1.5 channel of the invention in a biological sample of a human being suspected of having Brugada syndrome. In a preferred embodiment of the invention, said antibodies are directed against extracellular loops of the NaV 1.5 channel of (SEQ ID NO:1) and related isoforms.

In a preferred embodiment of the invention said human being may be either asymptomatic or at high risk for Brugada Syndrome due to family history, previous events of heart atrial and/or ventricular fibrillation, diabetes or obesity.

According to a preferred embodiment the in vitro method comprises the following steps:
a) contacting the biological sample with one or more antigens that specifically bind to the antibodies directed against NaV1.5 channel of (SEQ ID NO:1) and related isoforms; and
b) detecting the binding of the antigen to the antibodies in the biological sample.

Preferably said one or more antigens of step a) bind to the antibodies directed against extracellular loops of Na V1.5 channel of (SEQ ID NO:1) and related isoforms.

More preferably said antigens are fragments of at least 7 amino acids from the binding sites of extracellular loops of NaV1.5 channel. In a preferred embodiment said binding sites of extracellular loops of NaV1.5 channel are selected between the sequences SEQ ID NO:2-SEQ ID NO:14.

According to a preferred embodiment of the in vitro method of detection of antibodies against NaV1.5 channel and related isoforms of the invention, said antigens are labelled.

In a further preferred embodiment of the in vitro method of the invention the biological sample is selected from the group consisting of plasma, PBMCs, whole blood, serum and peripheral blood, or a combination thereof. Preferably, the biological sample is plasma and/or PBMCs. In a preferred embodiment of the in vitro method of detection of antibodies against NaV1.5 channel and related isoforms of the invention, the detection of the binding of the antigens to the antibodies is performed by ELISA or Western blotting.

It is a further object of the invention an antagonist of the binding of the antibodies directed against NaV 1.5 channel (SEQ ID NO:1) and related isoforms for use therapeutic agent in the treatment of Brugada syndrome. Preferably, said antibodies are directed against extracellular loops of NaV1.5 channel of (SEQ ID NO:1).

For example, steroids such as Prednisone, Methylprednisolone, and Dexamethasone, as well as medicament like Colchicine and Hydroxychloroquine (Plaquenil) may be used for the treatment of Brugada syndrome.

The present invention further contemplates a therapeutic agent specifically targeting the antibodies directed against the NaV 1.5 channel (SEQ ID NO:1) and related isoforms for use in the treatment of Brugada syndrome. Preferably, said antibodies are directed against extracellular loops of Na V1.5 channel of (SEQ ID NO:1) and related isoforms.

According to a preferred embodiment of the invention the therapeutic agent is characterized in that it comprises one or more antigenic fragments of the extracellular loops of the Nav 1.5 channel (SEQ ID NO:1) and related isoforms.

Preferably, the autoimmune therapeutic agent comprises sequences of at least about 10 amino acids from the extracellular loops of the NaV 1.5 channel, said extracellular loops being selected from the group of the sequences SEQ ID NO:2-SEQ ID NO: 14.

Another possible therapeutic approach contemplates targeting B cells thus disrupting the production of autoantibodies that can trigger inflammation mediated by immune complexes. Drugs, such as Rituximab, Ocrelizumab, Ofatumumab, and Inebilizumab which have already demonstrated efficacy in treating various immune and autoimmune diseases B-cells mediated may be useful for the therapeutic treatment.

Alternatively, it is contemplated a nanobody-based multivalent or multi-specific to target Ab-Nav1.5 in order to restore regular activity of NaV 1.5 channels. Additionally, these nanobodies will serve as valuable tools for in vivo imaging of the cardiac substrate affected by Brugada syndrome, allowing for effective monitoring.

The invention further contemplates the use of the antagonist or the autoimmune therapeutic agent in combination with another therapy for Brugada syndrome in order to achieve a more comprehensive and effective treatment.

The present invention is further directed to a kit for detecting antibodies against the NaV 1.5 channel (SEQ ID NO:1) and related isoforms in a biological sample, the kit comprising one or more antigens that specifically bind to the autoantibodies against NaV1.5 channel. Preferably, said one or more antigens specifically bind to the antibodies directed against extracellular loops of NaV1.5 channel of (SEQ ID NO:1). Even more preferably said antigens comprises one or more antigenic fragments or epitope belonging to the binding site of the extracellular loops of the NaV1.5 protein, said binding site of the extracellular loops being selected from the group of sequences SEQ ID NO:2-SEQ ID NO: 14.

In a preferred embodiment of the kit of the invention, said antigens are labelled or attached to a solid support. Preferably, said antibodies are labelled with fluorescents.

Said solid support is preferably a multi-well plate. Preferably, said kit is an ELISA kit.

The present invention further relates to an antigenic fragment or epitope belonging to a binding site of the extracellular loop of NaV 1.5 channel selected from the group consisting of the sequences SEQ ID NO:2-SEQ ID NO: 14.

Moreover, the invention is directed to the use of one or more of the autoantibodies against NaV 1.5 channel (SEQ ID NO:1) and related isoforms for the diagnosis of Brugada Syndrome in a human being by (qualitative or quantitative) detection of the presence thereof in a biological sample, wherein said biological sample is selected from the group consisting of plasma, PBMCs, whole blood, serum and peripheral blood, or a combination thereof. According to a preferred embodiment of the invention, said detection step is carried out by using one or more antigens that specifically binds to the autoantibodies against NaV 1.5 channel (SEQ ID NO:1) and related isoforms. In a further preferred embodiment of the invention said one or more antigens are selected from the antigenic fragments or epitopes belonging to a binding site of the extracellular loop of NaV 1.5 channel consisting of the sequences SEQ ID NO:2-SEQ ID NO:14.

Finally, the invention contemplates the use of one or more of the autoantibodies of the invention as marker for monitoring the efficacy of a therapeutic treatment of Brugada Syndrome disease by detection of the autoantibodies titer in a biological sample of a treated patient. Said biological sample may be selected from the group consisting of plasma, PBMCs, whole blood, serum and peripheral blood, or a combination thereof. According to a preferred embodiment said therapeutic treatment could be pharmacological or interventional-surgical, including ablation. The present invention will now be described, for non-limiting illustrative purposes, according to a preferred embodiment thereof, with particular reference to the attached figures, wherein:

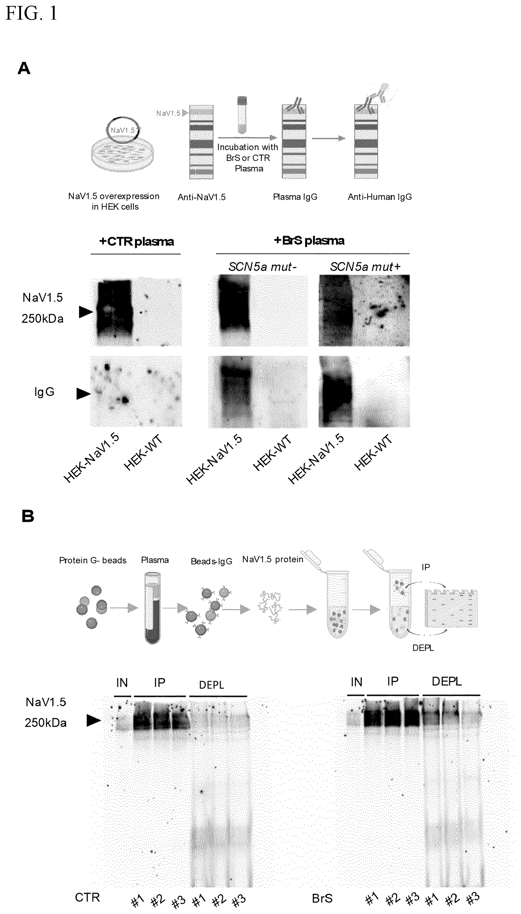

- Figure 1, shows autoantibodies detection against NaV1.5 channel in Brugada syndrome patient's plasma. Panel A) Western blot: proteins from lysed cells were separated in one-dimensional SDS-PAGE; NaV1.5 protein band was seen at 250-kd in lysates of transfected HEK cell (HEK-NaV1.5), but not in lysates of the non-transfected (HEK-WT) cells. Anti-rabbit Anti-NaV1.5 and anti-human-IgG probed the co-localization of IgGs from BrS patients with and without *SCN5A* mutations but not with healthy control. Panel B shows immunoprecipitation (IP) of NaV1.5 protein with IgG purified from plasma of BrS Patients and healthy control (n=3).

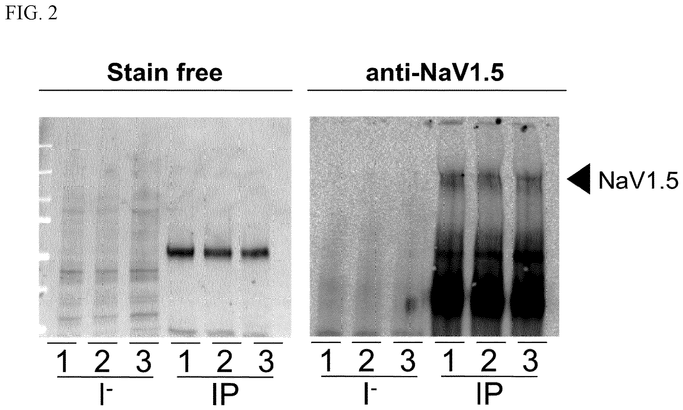

- Figure 2 shows Western blot analysis indicating that IgGs from Brugada Syndrome plasma are able to specifically bind NaV1.5 from protein lysate of cardiac mouse tissue.

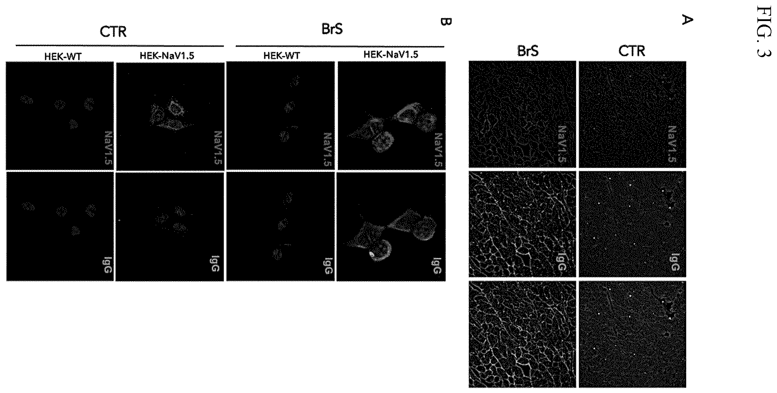

- Figure 3 shows representative fluorescence images validating the presence of NaV1.5 autoantibodies in plasma from patients with Brugada syndrome. Panel A) Representative fluorescence image displaying BrS positive and negative samples. Red fluorescence highlights NaV1.5 expression, while green fluorescence indicates the presence of bound human antibodies. The same plasma was tested on both transfected and non-transfected cells (HEK-WT), Panel B) Representative fluorescence image illustrating BrS positive and negative plasma. Red fluorescence indicates NaV1.5 expression, while green fluorescence serves as an indicator of human IgGs.

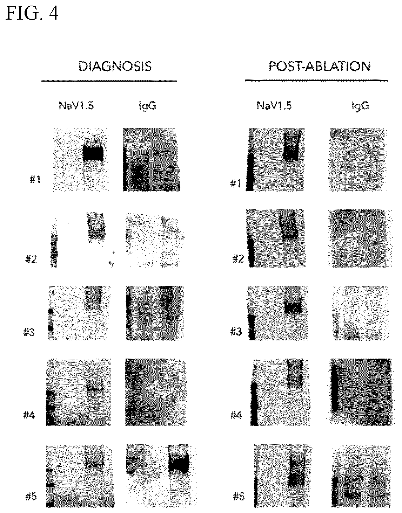

- Figure 4 shows detection of autoantibodies against NaV1.5 protein from plasma of patients with Brugada syndrome at the time of diagnosis (right panel) and after catheter ablation (left panel). Western blot analysis: proteins from lysed cells were separated using one-dimensional SDS-PAGE. A NaV1.5 protein band at 250-kd was observed in lysates from transfected HEK cells (HEK-NaV1.5) but not in lysates from untransfected cells (HEK-WT). Co-localization of IgGs from Brugada syndrome patients with and without *SCN5A* mutations, but not from Post Ablated (PA) patients was studied with anti-rabbit anti-NaV1.5 and anti-human IgG antibodies.

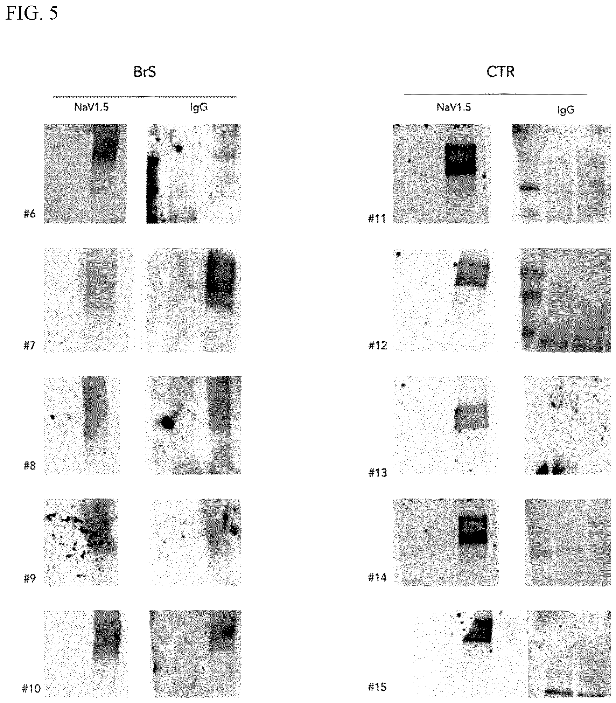

- Figure 5 shows detection of autoantibodies against NaV1.5 protein in the plasma of Brugada syndrome patients. Western blot analysis: proteins from lysed cells were separated by one-dimensional SDS-PAGE. A NaV1.5 protein band at 250-kd was observed in lysates from transfected HEK cells (HEK-NaV1.5) but not in lysates from untransfected cells (HEK-WT). Co-localization of IgGs from BrS patients with and without *SCN5A* mutations, but not from healthy controls, was studied with anti-rabbit anti-NaV1.5 and anti-human IgG antibodies.

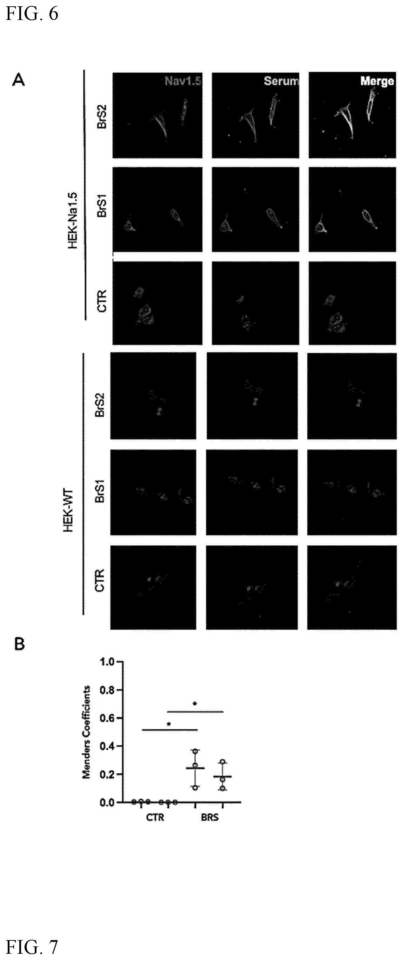

- Figure 6 shows a comparison of representative fluorescence image of Brugada syndrome positive and negative plasma. Panel A: Representative fluorescence image showing BrS positive and negative plasma. Red fluorescence indicates NaV1.5 expression, while green fluorescence serves as an indicator of human IgGs. Panel B): Menders coefficient.

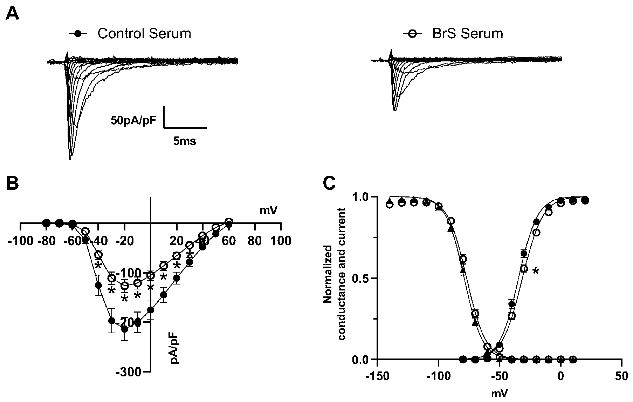

- Figure 7 shows effects of autoantibodies from Brugada Syndrome patients on sodium current. Panel A) Sodium current profiles in HEK293A cells overexpressing NaV1.5 incubated with either control (CTR, left panel) or Brugada Syndrome serum (BrS, right panel, filled circle). Panels B-C) Average current-voltage relationships (I-V) (N=6, n=70 for control, empty circle; N=8, n=91 for BrS serum, filled circle).

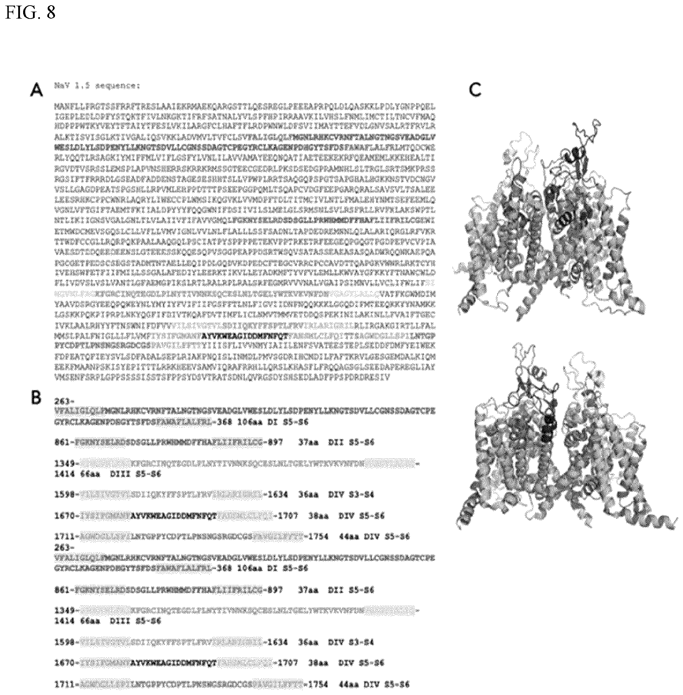

- Figure 8 shows the identification of putative binding sites of autoantibodies on NaV1.5 protein. Panel A) Amino acid sequence of NaV1.5 channel (SEQ ID NO:1); Panel B) Identification of six potential regions that serve as binding sites for autoantibodies. The 10 amino acids critical for binding on the solid support are highlighted in yellow; Panel C) PyMol structure showing the core of the NaV1.5 channel and the extracellular loop.

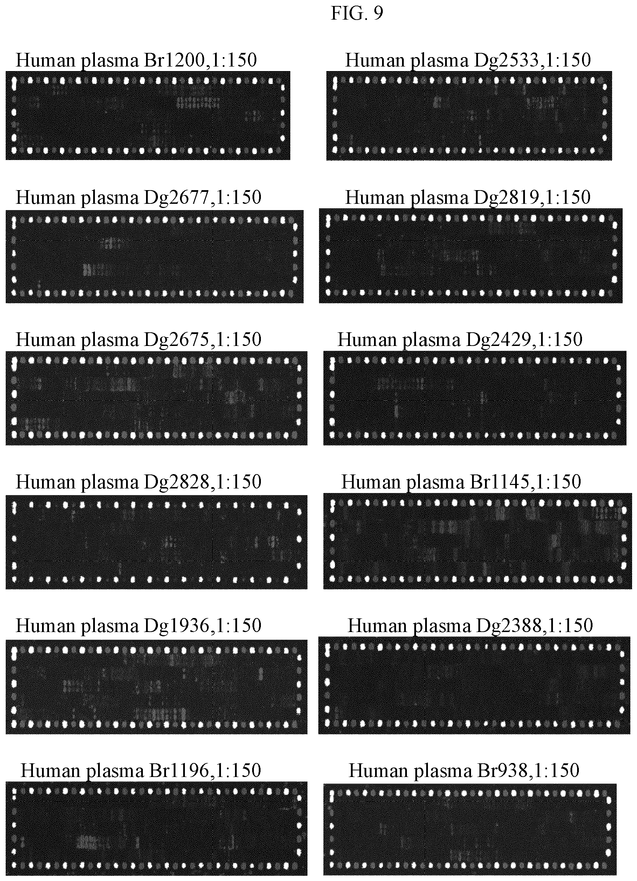

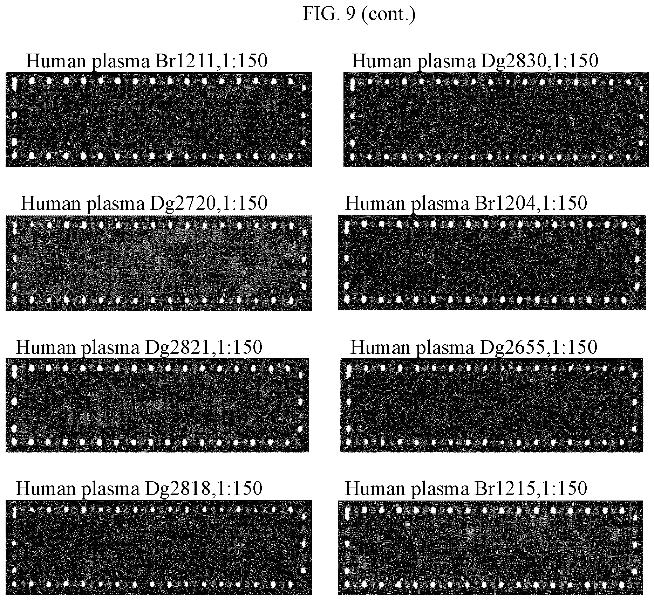

- Figure 9 shows peptide microarray scans of the human plasma of 20 patients with Brugada syndrome (Br1200, Dg2675, Dg1936, Br1211, Dg2821, Dg2533, Dg2429, Dg2388, Dg2830, Dg2655, Dg2677, Dg2828, Br1196, Dg2720, Dg2818, Dg2819, Br1145, Br938, Br1204, Br1215) analyzed.

The following examples are merely illustrative and should not be considered limiting the scope of the present invention.

### EXAMPLE 1: Detection of NAV1.5 channel autoantibodies in plasma from Brugada patients

### METHODS

### Human samples

Brugada patients diagnosed with Brugada Syndrome (BrS) at the Arrhythmology Department of IRCCS Policlinico San Donato were included in this study [24]. Participants were classified into three main subgroups: 1) BrS patients with *SCN5A* mutations; 2) BrS patients without *SCN5A* variants; and 3) healthy controls. Additionally, five patients underwent catheter ablation. The study protocol was approved by the local Institutional Ethics Committee, and written informed consent was obtained from all participants in accordance with the Declaration of Helsinki (NCT02641431; NCT03106701) [25, 26]. All patients met the diagnostic criteria for BrS, including the presence of a spontaneous or drug-induced type 1 Brugada ECG pattern. Clinical data, medical history, 12-lead ECG recordings, and implantable cardioverter-defibrillator (ICD) outcomes were collected from medical records.

### Cell culture

HEK293 cells were cultured in DMEM high-glucose medium supplemented with 10% fetal bovine serum, 1% penicillin-streptomycin, and 1% glutamine at 37°C under 5% CO₂.

### Mouse Model

Adult C57BL-6 mice were housed under standard conditions with a 12-hour light/dark cycle and ad libitum access to food and water. The mice were kept in the same controlled environment.

### Whole Exome Sequencing

All patients were studied using a Next Generation Sequencing panel of genes, including *SCN5A,* from peripheral blood extracted DNA. The DNA was extracted from peripheral blood and processed to obtain libraries containing approximately 575 kb of genomic DNA, using 50 nanograms of DNA input quantity. The libraries were subjected to deep sequencing, and after removing duplicates and filtering low-quality reads, an average target coverage of 100X was achieved. Sanger sequencing was used to validate the Next Generation Sequencing data, following the guidelines of the American College of Medical Genetics (ACMG) [27]. Variants were annotated with information from well-known public databases, including dbSNP, dbNSFP, ExAC, and ClinVar.

### Plasma Collection and Processing

Blood (25 ml) was centrifuged at 1000g for 15 minutes to isolate plasma, followed by centrifugation of the supernatant at 2000g for 15 minutes. The supernatant was collected, aliquoted, and stored at -20°C.

### IgG Isolation

IgG antibodies were isolated using the PureProteome^{™} Protein G Magnetic Bead System. The isolation was performed under non-denaturing conditions with high salt concentration and nearly neutral pH.

### Stable cell line generation and transient transfections

To express the NaV1.5 channel protein, a full-length cDNA encoding human *SCN5A* was synthesized and cloned into pcDNA 3.1(+). HEK293 cells were transfected with the pcDNA 3.1(+)/*SCN5A* construct using ViaFect transfection reagent (Promega) according to the manufacturer's instructions. Cells were growth in medium consisting of Dulbecco's Modified Eagle Medium (DMEM, Life Technologies) supplemented with 10% fetal bovine serum (FBS, Sigma), 2 mM glutamine (Merck), and 1X penicillin/streptomycin (Euroclone) and selected with G418 at a specified concentration. The cells were maintained at 37°C in a 5% CO2 and 95% air-humidified atmosphere. Harvested cells were lysed, and the cleared lysate was collected after centrifugation. The successful transfection and expression of NaV1.5 channels were confirmed through Western blot analysis.

### Western Blot

Cells were lysed in a lysis buffer containing 150 mM NaCl, 50 mM Tris-HCl pH 7.5, 1% Triton X-100, 0.5% sodium deoxycholate, and 0.1% SDS, supplemented with protease and protein phosphatase inhibitors. Total protein concentration was measured using the BCA assay. Proteins were denatured and reduced in a Laemmli-b-mercaptoethanol mixture at 100°C for 5 min. Subsequently, proteins (30 µg) were loaded onto a 10% SDS-PAGE gel and transferred to a nitrocellulose membrane. Nonspecific binding was blocked, and the membrane was incubated overnight at 4°C with a primary rabbit monoclonal anti-NaV1.5 antibody (1:2000 dilution, clone D9J7S, Cell Signalling). After washing, the membrane was incubated with an appropriate anti-rabbit-Alexafluoor546-conjugated secondary antibody (1:2000 dilution) for 1 hour at room temperature. Following further washes, the membrane was incubated with plasma from patients or controls diluted 1:3 in PBS for 2 hours at room temperature. After additional washes, a secondary antibody for human IgG-FITC was added for 1 hour at room temperature. Immunoreactive bands were visualized using an enhanced chemiluminescence detection kit (ECL Advance, GE Healthcare).

### NaV1.5 channel Immunoprecipitation from protein lysate of cardiac mouse tissue

To collect the heart samples, the animals were anesthetized by intraperitoneal injection of medetomidine, 0.5 mg/kg (Orion Pharma S.r.l.) and ketamine, 100 mg/kg (Merial), both diluted in saline. Once the animals were completely unconscious, their thorax was opened, and the heart was perfused with 1 ml KCl 1M to induce diastolic arrest and flushed with 0.9% saline through a cannula inserted into the left ventricle. The heart was then excised, and the left ventricle was separated from the atria and right ventricle. The left ventricle was then divided into 3-mm-thick slices using a specific stainless steel heart matrix (Roboz Surgical Instruments) to create apex, middle, and base specimens. The apex was incubated with 500 ml RIPA Lysis Buffer and homogenized using the tissue homogenizer Lyser^{®} (Qiagen). Each sample was subjected to three homogenization cycles, each lasting 5 minutes at a rate of 25 oscillations per second. The homogenate was kept in ice for 30 minutes and then centrifuged at 10000 rcf for 10 minutes at 4°C. After centrifugation, the supernatant of each tissue sample was transferred to a new tube, and the total protein content was quantified using the BCA Protein Assay Kit (Thermo Fisher Scientific) according to the manufacturer's instructions.

300 µg of total protein were incubated with Dynabeads^{™} Protein G bound with IgGs from BrS plasma or control, and the eluted fraction was subsequently subjected to western blot analysis stained for NaV1.5, as previously described.

### NaV1.5 Immunoprecipitation from transfected HEK-293 cells

Immunoprecipitation of the NaV1.5 channel protein from transfected HEK-293 cells was performed using IgG from serum of patients and healthy controls specific for NaV1.5 channel. The immunoprecipitation was carried out by incubating the antibodies with Dynabeads Protein G for 30 minutes at room temperature. After washing, the beads were incubated with total cell lysate for 2 hours at room temperature with orbital shaking. Following additional washes, Laemmli-b-mercaptoethanol sample buffer was added to the beads, and the eluted fraction was subjected to SDS-PAGE.

### Immunofluorescence on Mouse Tissue

Immunofluorescence assays were conducted on adult C57BL-6 mouse heart tissue. Left ventricle sections, 12 µm thick, were prepared using a cryostat and mounted onto gelatin-coated histological slides. The sections were thawed, rehydrated, and subjected to antigen unmasking. Non-specific binding was blocked using a solution containing normal donkey serum and bovine serum albumin. Incubation with patient plasma diluted 1:50 in PBS with 2% NDS and 2% BSA was performed, followed by washing and incubation with an anti-human IgG-FITC secondary antibody. Subsequently, the sections were incubated with a primary rabbit monoclonal anti-NaV1.5 antibody (1:200 dilution; clone D9J7S, Cell Signaling), followed by washing and incubation with an appropriate anti-rabbit secondary antibody conjugated with Cy3. After further washes, the sections were mounted with Vectashield mounting medium containing DAPI. Images were captured using a Leica Thunder microscope (×40 objective).

### Cell-based assay immunofluorescence (CBA-IF)

To assess the binding of IgGs-NaV1.5 autoantibodies, live HEK293 WT and HEK293 cells expressing NaV1.5 protein were used. The cells were plated on coverslips and incubated with patient plasma diluted 1:3 in DMEM HG supplemented with 1% L-glutamine, 1% penicillin-streptomycin, and 5% heat-inactivated serum. After washing, the cells were fixed in cold methanol and subjected to blocking. Incubation with NaV1.5 antibody was performed, followed by washing and staining with a rabbit-secondary antibody and FITC anti-human IgG. Nuclear staining was performed using DAPI. Images were captured using a Leica Thunder microscope (×40 objective).

### Colocalization quantification

Confocal microscopy images were randomly captured for all groups, and colocalization analysis was performed using the JACoP plugin in the FIJI software. The Manders' coefficient was calculated to quantify colocalization.

### Cellular Electrophysiology

The chip-based automated planar patch-clamp system Patchliner (Nanion Technologies GmbH, Munchen, Germany) was employed to record sodium currents from HEK293A cells transiently transfected with the NaV1.5 channel. After 1 hour incubation at 37°C and 5% CO₂ with 5% BrS or Control derived serum, cells were gently tripsinized and resuspended in the extracellular low-sodium recording solution from Nanion (Ref. 08-3004, ionic composition in mM: 80 NaCl, 60 NMDG, 4 KCl, 2 CaCl₂, 1 MgCl₂, 5 D-Glucose monohydrate, 10 Hepes; pH 7.4 with HCl, 289 mOsm). Non treated cells were used as internal reference. In order to improve membrane stability, cells were then incubated at 4°C for 20 minutes. All recordings were performed at room temperature in whole-cell configuration using medium resistance NPC-16 chips. At least two chips for condition were used on each experimental day. CsF-based intracellular (Ref. 08-3008, ionic composition in mM: 10 EGTA, 10 Hepes, 10 CsCl, 10 NaCl, 110 CsF; pH 7.2 with CsOH, 280 mOsm) and the extracellular seal enhancer (Ref. 08-3011, ionic composition in mM: 80 NaCl, 60 NMDG, 4 KCl, 10 CaCl2, 1 MgCl₂, 5 D-Glucose monohydrate, 10 Hepes; pH 7.4 with HCl, 313 mOsm) solutions were also provided from Nanion. In order to reduce any bias due to transfection variability, at least one BrS serum and one Control serum from healthy donors were tested on each individual experimental day. Cell capacitance and series resistance were automatically compensated by the Patchliner. All currents were sampled at 50 kHz. The current-voltage (I-V) relationship and the voltage-dependence of activation of the sodium currents were obtained applying a protocol with incremental 50 ms steps ranging from -80 to +60 mV (holding potential -120 mV). Raw traces recorded by HEK293A amplifiers were exported with a home-built Python tool and individual traces were analysed using Clampfit 10.7 (Molecular Devices, San Jose, CA, USA), Origin Pro (OriginLab, Norhampton, MA, USA), and GraphPad Prism (GraphPad Software, Boston, MA, USA). Current density was calculated dividing the current amplitude (pA) by the cell capacitance (pF) for each cell.

### Prediction of NaV1.5 autoantibody binding-site on NaV1.5 channel protein

To explore binding sites on the NaV1.5 channel protein for autoantibodies, molecular modeling was conducted using the crystal structure of the NaV1.5 channel protein downloaded from Uniprot *SCN5A* (Figure 6). Pymol software was employed to predict favorable interactions between the autoantibodies and the target protein, facilitating the rational design of binding sites.

### Epitope discovery

The sequences of loops 1-6 of NaV 1.5 (DI S5-S6 (263-368), DII S5-S6 (861-897), DIII S5-S6 (1349-1414), DIV S3-S4 (1598-1634), DIV S5-S6 (1670-1707) & DIV S5-S6 (1711-1754)) were elongated with neutral GSGSGSG linkers at the N- and C-termini to avoid truncated peptides. The elongated sequences were converted into 15 amino acid peptides with a peptide-peptide overlap of 14 amino acids. The resulting NaV 1.5 peptide microarrays contained 327 different peptides printed in duplicate (654 peptide spots) and were framed by additional HA (YPYDVPDYAG, 40 spots) and polio (KEVPALTAVETGAT, 38 spots) control peptides. The microarray was subjected to Rockland blocking buffer MB-070 for 30 min and then pre-stained with the secondary antibody goat anti-human IgG (Fc) DyLight680 (0.1 µg/ml) for 45 min staining in incubation buffer at RT to investigate background interactions that could interfere with the main assays.

Incubation of further NaV 1.5 peptide microarrays with 20 Brugada syndrome patients plasma (Br1200, Dg2675, Dg1936, Br1211, Dg2821, Dg2533, Dg2429, Dg2388, Dg2830, Dg2655, Dg2677, Dg2828, Br1196, Dg2720, Dg2818, Dg2819, Br1145, Br938, Br1204, Br1215) at a dilution of 1:150 incubation buffer (PBS, pH 7.4 with 0.05% Tween 20; washing for 3 × 10 sec with 10% of Rockland blocking buffer MB-070) for 16 h at 4°C and orbital shaking at 140 rpm was followed by staining with the secondary antibody.

Read-out was performed with an Innopsys InnoScan 710-IR Microarray Scanner. The additional HA peptides framing the peptide microarrays were simultaneously stained with mouse monoclonal anti-HA (12CA5) DyLight800 (0.2 µg/ml) for 45 min in (PBS, pH 7.4 with 0.05% Tween 20; washing for 3 × 10 sec with 10% of Rockland blocking buffer MB-070) at RT.

Quantification of spot intensities and peptide annotation were based on 16-bit gray scale tiff files that exhibit a higher dynamic range than the 24-bit colorized tiff files shown in this report.

Microarray image analysis was done with PepSlide^{®} Analyzer a software algorithm breaks down fluorescence intensities of each spot into raw, foreground and background signals, and calculates averaged median foreground intensities. Based on averaged median foreground intensities, intensity maps were generated and interactions in the peptide maps highlighted by an intensity color code with red for high and white for low spot intensities. Innopsys InnoScan 710-IR Microarray Scanner was used with resolution of 20 µm; scanning gains of 50 at low laser power (680 nm, red) and 10 at high laser power (800 nm, green).

### RESULTS

### Autoantibody Detection Against NaV1.5 Protein in Brugada syndrome plasma

A cohort of 100 subjects was included in the study. Western blot analysis was initially conducted to detect the presence of IgG autoantibodies against the NaV1.5 channel in plasma samples from the patients. Among the BrS patients tested positive for the Ajmaline test (n = 53), 48 patients without *SCN5A* mutations and 5 patients carrying *SCN5A* variants exhibited the autoantibody. In contrast, plasma samples from 47 subjects who tested negative in the Ajmaline test showed no presence of the autoantibody (Figure 1A and Figure 5). To further confirm the presence of IgG against the NaV1.5 channel, immunoprecipitation assays were performed. IgGs isolated from the plasma of patients positive for the autoantibody exhibited binding to NaV1.5 protein (Figure 1B) extracted from HEK cell lysate.

### Mouse NaV1.5 is immunoprecipitated by BrS anti-NaV1.5 autoantibodies

Western blot analysis indicate that IgGs from BrS plasma are able to specifically bind NaV1.5 from protein lysate of cardiac mouse tissue (Figure 2).

Magnetic beads coated with IgGs from BrS patients were employed to incubate with mouse cardiac ventricular protein extract. Subsequently, the immunoprecipitated (IP) and immunodepleted (I-) fractions were resolved through Western blot analysis (left blot).

The presence of NaV1.5 protein in the IP fraction, as revealed by staining with a commercial anti-NaV1.5 antibody (right blot), confirmed the existence of anti-NaV1.5 IgGs in BrS plasma.

### Binding of NaV1.5 by Plasma IgG from BrS Patients on Cells Over-expressing NaV1.5

### Channel

To investigate the binding of NaV1.5 by plasma IgG from BrS patients, double immunofluorescence labelling was performed on mouse heart slides using an anti-NaV1.5 monoclonal antibody and plasma from BrS patients and healthy controls. Colocalization of the immunofluorescence signal was observed when the monoclonal anti-NaV1.5 antibody and plasma from BrS patients were used, while no signal was observed with healthy control plasma (Figure 3A). Similar results were obtained with immunolabeling on cells over-expressing the NaV1.5 channel using plasma from BrS patients. The anti-NaV1.5 antibodies from BrS patients specifically bound to NaV1.5-transfected cells, but not mock-transfected cells (Figure 3B and Figure 6).

### Undetectable Autoantibody against NaV1.5 in BrS Patients following Catheter Ablation

To assess the impact of catheter ablation on the presence of autoantibodies against NaV1.5, a subset of five patients from the initial cohort was analyzed after six months from the ablation procedure. Western blot analysis showed an absence of IgG autoantibodies against NaV1.5 in these patients following epicardium ablation (Figure 4).

### Effects of autoantibodies from Brugada Syndrome patients on sodium current.

Figure 7 shows that autoantibodies from Brugada Syndrome patients decrease sodium current. Automated patch-clamp in whole-cell configuration was used to record sodium current in HEK293 cells overexpressing NaV1.5 channel, incubated for 1 hour with either 5% BrS patient- or healthy donor- plasma. Results showed a significant ≈40% reduction in current density in in the former, with a mean peak current density at -20 mV was -122.3±12 pA/pF (n=100), compared to -211.2±21 pA/pF (n=79) measured in the latter condition. As internal reference, the current density recorded in non-treated HEK293 overexpressing NaV1.5 was 169.8±12 (n=54), not different from the current density obtained in cells incubated with the healthy donor's plasma. Data indicate a significant reduction (approximately 40%) in inward sodium current with Brugada syndrome plasma exposure compared with control plasma.

### Prediction of Putative NaV1.5 Autoantibody Binding-Sites on NaV1.5 Channel Protein

The human NaV1.5 channel has the following amino acid sequence:

(SEQ ID NO:1)







































Using molecular modelling, the authors identified six (DI-DVI) putative binding sites for autoantibodies on the NaV1.5 channel protein extracellular loops (in bold in SEQ ID NO:1) as represented in Figure 8:
**DI S5-S6 (263-368) 106 aa:** 
VFALIGLQLFMGNLRHKCVRNFTALNGTNGSVEADGLVWESLDLYLSDPENYLLKN
GTSDVLLCGNSSDAGTCPEGYRCLKAGENPDHGYTSFDSFAWAFLALFRL (SEQ ID
NO:2)
**DII S5-S6 (861-897) 37 aa:**
   FGKNYSELRDSDSGLLPRWHMMDFFHAFLIIFRILCG (SEQ ID NO:3)
**DIII S5-S6 (1349-1414) 66 aa** 
SIMGVNLFAGKFGRCINQTEGDLPLNYTIVNNKSQCESLNLTGELYWTKVKVNFDNV
GAGYLALLQ (SEQ ID NO: 4)
**DIV S3-S4 (1598-1634) 36 aa** VILSIVGTVLSDIIQKYFFSPTLFRVIRLARIGRIL (SEQ ID NO:5)
**DV S5-S6 (1670-1707) 38 aa** IYSIFGMANFAYVKWEAGIDDMFNFQTFANSMLCLFQI (SEQ ID NO:6)
**DVI S5-S6 (1711-1754) 44aa** AGWDGLLSPILNTGPPYCDPTLPNSNGSRGDCGSPAVGILFFTT (SEQ ID NO:7)

### Epitope identification in human plasma of Brugada patients

Incubation of the NaV 1.5 peptide microarray with human plasma Dg2677, Dg2818, Br1200, Dg2675, Dg1936, Br1211, Dg2821, Dg2533, Dg2429, Dg2388, Dg2830, Dg2655, Dg2828, Br1196, Dg2720, Dg2819, Br1145, Br938, Br1204, Br1215 at a dilution of 1:150 was followed by staining with the secondary and control antibodies (data not shown).

The incubation of NaV 1.5 peptide microarrays with 20 Brugada syndrome patients plasma (Figure 9) leads to the identification of seven binding sites for autoantibodies on the NaV1.5 channel protein (SEQ ID NO:1) characterized by the following sequences:
DI-S5-S6 (263-368): SVEADGLVWESLDLYLSDPENYLLKNGTS (SEQ ID NO:8)
DI S5-S6 (263-368): CLKAGENPDHGYTSFDSFAWAFLAL (SEQ ID NO:9)
DIII S5-S6 (1349-1414): DSDSGLLPRWHMMDFFHAFLII (SEQ ID NO:10)
DIII S5-S6 (1349-1414): VNNKSQCESLNLTGEYWTKVK (SEQ ID NO:11)
DIV S3-S4 (1598-1634): GSGVILSIVGTVLSDIIQKYFFSPT (SEQ ID NO:12)
DIV S5-S6 (1670-1707): MANFAYVKWEAGIDDMFNFQTFANSMLCLF (SEQ ID NO:13)
DIV S5-S6 (1711-1754): GWDGLLSPILNTGPPYCDPTLPNSNGSRGD (SEQ ID NO:14).

### REFERENCES

1. Antzelevitch, C. and B. Patocskai, Brugada Syndrome: Clinical, Genetic, Molecular, Cellular, and Ionic Aspects. Curr Probl Cardiol, 2016. 41(1): p. 7-57.
2. Nademanee, K., et al., Fibrosis, Connexin-43, and Conduction Abnormalities in the Brugada Syndrome. J Am Coll Cardiol, 2015. 66(18): p. 1976-1986.
3. Priori, S.G., et al., 2015 ESC Guidelines for the management of patients with ventricular arrhythmias and the prevention of sudden cardiac death: The Task Force for the Management of Patients with Ventricular Arrhythmias and the Prevention of Sudden Cardiac Death of the European Society of Cardiology (ESC). Endorsed by: Association for European Paediatric and Congenital Cardiology (AEPC). Eur Heart J, 2015. 36(41): p. 2793-2867.
4. Batchvarov, V.N., The Brugada Syndrome - Diagnosis, Clinical Implications and Risk Stratification. Eur Cardiol, 2014. 9(2): p. 82-87.
5. Ghiroldi, A., et al., Alterations of the Sialylation Machinery in Brugada Syndrome. Int J Mol Sci, 2022. 23(21).
6. Keller, D.I., et al., Brugada syndrome and fever: genetic and molecular characterization of patients carrying SCN5A mutations. Cardiovasc Res, 2005. 67(3): p. 510-9.
7. Bare, J., et al., Genome-wide association analyses identify new Brugada syndrome risk loci and highlight a new mechanism of sodium channel regulation in disease susceptibility. Nat Genet, 2022. 54(3): p. 232-239.
8. Sonoda, K., et al., Copy number variations of SCN5A in Brugada syndrome. Heart Rhythm, 2018. 15(8): p. 1179-1188.
9. Wijeyeratne, Y.D., et al., SCN5A Mutation Type and a Genetic Risk Score Associate Variably With Brugada Syndrome Phenotype in SCN5A Families. Circ Genom Precis Med, 2020. 13(6): p. e002911.
10. Frustaci, A., et al., Cardiac histological substrate in patients with clinical phenotype of Brugada syndrome. Circulation, 2005. 112(24): p. 3680-7.
11. Corrado, D., et al., Relationship Between Arrhythmogenic Right Ventricular Cardiomyopathy and Brugada Syndrome: New Insights From Molecular Biology and Clinical Implications. Circ Arrhythm Electrophysiol, 2016. 9(4): p. e003631.
12. Miles, C., et al., Biventricular Myocardial Fibrosis and Sudden Death in Patients With Brugada Syndrome. J Am Coll Cardiol, 2021. 78(15): p. 1511-1521.
13. Pieroni, M., et al., Electroanatomic and Pathologic Right Ventricular Outflow Tract Abnormalities in Patients With Brugada Syndrome. J Am Coll Cardiol, 2018. 72(22): p. 2747-2757.
14. Chatterjee, D., et al., An autoantibody identifies arrhythmogenic right ventricular cardiomyopathy and participates in its pathogenesis. Eur Heart J, 2018. 39(44): p. 3932-3944.
15. Meraviglia, V., et al., Inflammation in the Pathogenesis of Arrhythmogenic Cardiomyopathy: Secondary Event or Active Driver? Front Cardiovasc Med, 2021. 8: p. 784715.
16. Patel, P.A. and A.F. Hernandez, Targeting anti-beta-1-adrenergic receptor antibodies for dilated cardiomyopathy. Eur J Heart Fail, 2013. 15(7): p. 724-9.
17. Labovsky, V., et al., Anti-beta1-adrenergic receptor autoantibodies in patients with chronic Chagas heart disease. Clin Exp Immunol, 2007. 148(3): p. 440-9.
18. Li, J., et al., Anti-KCNQ1 K(+) channel autoantibodies increase IKs current and are associated with QT interval shortening in dilated cardiomyopathy. Cardiovasc Res, 2013. 98(3): p. 496-503.
19. Korkmaz, S., et al., Provocation of an autoimmune response to cardiac voltage-gated sodium channel NaV1.5 induces cardiac conduction defects in rats. J Am Coll Cardiol, 2013. 62(4): p. 340-9.
20. Chatterjee, D., et al., An autoantibody profile detects Brugada syndrome and identifies abnormally expressed myocardial proteins. Eur Heart J, 2020. 41(30): p. 2878-2890.
21. Mader, S., et al., Complement activating antibodies to myelin oligodendrocyte glycoprotein in neuromyelitis optica and related disorders. J Neuroinflammation, 2011. 8: p. 184.
22. Duong, S.L. and H. Pruss, Molecular disease mechanisms of human antineuronal monoclonal autoantibodies. Trends Mol Med, 2023. 29(1): p. 20-34.
23. Kobayashi, S., et al., Autoantibody-induced internalization of nicotinic acetylcholine receptor alpha3 subunit exogenously expressed in human embryonic kidney cells. J Neuroimmunol, 2013. 257(1-2): p. 102-6.
24. Pappone, C. and V. Santinelli, Brugada Syndrome: Progress in Diagnosis and Management. Arrhythm Electrophysiol Rev, 2019. 8(1): p. 13-18.
25. Pappone, C., et al., Assessing the Malignant Ventricular Arrhythmic Substrate in Patients With Brugada Syndrome. J Am Coll Cardiol, 2018. 71(15): p. 1631-1646.
26. Pappone, C., et al., Electrical Substrate Elimination in 135 Consecutive Patients With Brugada Syndrome. Circ Arrhythm Electrophysiol, 2017. 10(5): p. e005053.
27. Richards, S., et al., Standards and guidelines for the interpretation of sequence variants: a joint consensus recommendation of the American College of Medical Genetics and Genomics and the Association for Molecular Pathology. Genet Med, 2015. 17(5): p. 405-24.

## Claims

1. Antibodies for use as biomarkers for the diagnosis of Brugada Syndrome in a human being, said antibodies being directed against NaV 1.5 channel (SEQ ID NO:1) and related isoforms.

2. Antibodies for use according to claim 1, directed against a binding site of the extracellular loops of NaV 1.5 channel (SEQ ID NO:1) and related isoforms.

3. Antibodies for use according to claim 2, wherein said binding site of the extracellular loops of NaV 1.5 channel and related isoforms is selected from the group comprising the following the sequences: 
i) VFALIGLQLFMGNLRHKCVRNFTALNGTNGSVEADGLVWESLDLYLS
DPENYLLKNGTSDVLLCGNSSDAGTCPEGYRCLKAGENPDHGYTSFDSFAWAFLALF
RL (SEQ ID NO:2)
ii) FGKNYSELRDSDSGLLPRWHMMDFFHAFLIIFRILCG (SEQ ID NO:3)
iii) SIMGVNLFAGKFGRCINQTEGDLPLNYTIVNNKSQCESLNLTGELYW
TKVKVNFDNVGAGYLALLQ-1414 (SEQ ID NO: 4)
iv) VILSIVGTVLSDIIQKYFFSPTLFRVIRLARIGRIL (SEQ ID NO:5)
v) IYSIFGMANFAYVKWEAGIDDMFNFQTFANSMLCLFQI (SEQ ID NO:6)
vi) AGWDGLLSPILNTGPPYCDPTLPNSNGSRGDCGSPAVGILFFTT (SEQ ID NO:7)
vii) SVEADGLVWESLDLYLSDPENYLLKNGTS (SEQ ID NO:8)
viii) CLKAGENPDHGYTSFDSFAWAFLAL (SEQ ID NO:9)
ix) DSDSGLLPRWHMMDFFHAFLII (SEQ ID NO:10)
x) VNNKSQCESLNLTGEYWTKVK (SEQ ID NO:11)
xi) GSGVILSIVGTVLSDIIQKYFFSPT (SEQ ID NO:12)
xii) MANFAYVKWEAGIDDMFNFQTFANSMLCLF (SEQ ID NO:13)
xiii) GWDGLLSPILNTGPPYCDPTLPNSNGSRGD (SEQ ID NO:14) or fragments thereof.

4. An in vitro method for detecting the presence or detecting the amount of at least one of the antibodies directed against the NaV 1.5 channel (SEQ ID NO:1) and related isoforms according to anyone of the claim 1-3, in a biological sample of a human being suspected of having Brugada syndrome.

5. An in vitro method according to claim 4, which comprises the following steps:
a) contacting the biological sample with one or more antigens that specifically binds to the antibodies directed against NaV 1.5 channel (SEQ ID NO:1) and related isoforms according to anyone of claims 1-3;
and
b) detecting the binding of the antigen to the antibodies in said biological sample.

6. An in vitro method according to anyone of claims 4-5, wherein said one or more antigens are labelled.

7. An in vitro method to anyone of claims 4-6, wherein said antigens are fragments of at least 10 amino acids from the binding sites of extracellular loops of NaV1.5 channel.

8. An in vitro method according to claim 7, wherein said binding sites of extracellular loops of NaV1.5 channel are selected between SEQ ID NO:2-SEQ ID NO:14.

9. An in vitro method according to anyone of claims 4-8, wherein the biological sample is selected from the group consisting of plasma, PBMCs, whole blood, serum and peripheral blood, or a combination thereof.

10. An in vitro method according to anyone of claims 4-9, wherein the detection of the binding of said one or more antigens to the antibodies is performed by ELISA or Western blotting.

11. An antagonist of the binding of the antibodies according to anyone of claims 1-3 to the NaV 1.5 channel (SEQ ID NO:1) and related isoforms, preferably to one or more antigenic fragments of its extracellular loops, for use as therapeutic agent in the treatment of Brugada syndrome.

12. A therapeutic agent specifically targeting or disrupting the production of auto antibodies directed to the NaV 1.5 channel (SEQ ID NO:1) according to anyone of claims 1-3, for use in the treatment of Brugada syndrome.

13. A therapeutic agent for use in the treatment of Brugada syndrome according to claim 12 specifically targeting the autoantibodies directed to the NaV 1.5 channel (SEQ ID NO:1) and related isoforms **characterized in that** it comprises one or more antigenic fragments of the extracellular loops of the Nav 1.5 protein.

14. A therapeutic agent according to claim 13, **characterized in that** it comprises sequences of at least about 10 amino acids from the extracellular loops of the NaV 1.5 protein, said extracellular loops being selected from the group of SEQ ID NO:2-SEQ ID NO:14.

15. The antagonist or the therapeutic agent according to anyone of claims 11-14, for use in combination with another therapy for Brugada Syndrome.

16. A kit for detecting antibodies against NaV 1.5 channel in a biological sample, the kit comprising one or more antigens that specifically binds to the antibodies against NaV 1.5 channel (SEQ ID NO:1) and related isoforms according to anyone of claims 1-3.

17. A kit according to claim 16, wherein said antigens comprises one or more antigenic fragments of the extracellular loops of the Nav 1.5 protein, said antigenic fragments of the extracellular loops being selected from the group of sequences SEQ ID NO:2-SEQ ID NO: 14.

18. A kit according to anyone of claims 16-17, wherein said antigens are labelled or attached to a solid support.

19. An antigenic fragment or epitope belonging to a binding site of the extracellular loop of NaV 1.5 channel and related isoforms selected from the group comprising the following the sequences SEQ ID NO:2-SEQ ID NO: 14.

20. Use of one or more of the antibodies against NaV 1.5 channel (SEQ ID NO:1) and related isoforms according to anyone of the claims 1-3, for the diagnosis of Brugada Syndrome in a human being by detection of the presence thereof in a biological sample, wherein said biological sample is selected from the group consisting of plasma, PBMCs, whole blood, serum and peripheral blood, or a combination thereof.

21. Use according to claim 20, wherein said detection is carried out by using one or more antigens that specifically binds to the autoantibodies against NaV 1.5 channel (SEQ ID NO:1) and related isoforms.

22. Use according to claim 21, wherein said one or more antigens are selected from the antigenic fragments or epitopes belonging to a binding site of the extracellular loop of NaV 1.5 channel and related isoforms selected from the group comprising the following the sequences SEQ ID NO:2-SEQ ID NO: 14.

23. Use of one or more of the antibodies according to anyone of the claims 1-3, as marker for monitoring the efficacy of a therapeutic treatment of Brugada Syndrome disease by detection of the autoantibodies titer in a biological sample of a treated patient.

24. Use of one or more of the antibodies according to claim 23, wherein said therapeutic treatment is pharmacological or interventional-surgical, including ablation.
